(19) Europäisches Patentamt
European Patent Office
Office européen des brevets

(11) Veröffentlichungsnummer: **0 416 364 A1**

# (12) EUROPÄISCHE PATENTANMELDUNG

(21) Anmeldenummer: **90115969.9**

(51) Int. Cl.[5]: **C07C 333/04**

(22) Anmeldetag: **21.08.90**

(30) Priorität: **02.09.89 DE 3929169**

(43) Veröffentlichungstag der Anmeldung: **13.03.91 Patentblatt 91/11**

(84) Benannte Vertragsstaaten: **DE ES FR IT**

(71) Anmelder: **BAYER AG**

**W-5090 Leverkusen 1 Bayerwerk(DE)**

(72) Erfinder: **Bohnenpoll, Martin, Dr.**
**Hahnenweg 3**
**W-5000 Köln 80(DE)**
Erfinder: **Schubart, Rüdiger, Dr.**
**An der Engelsfuhr 27**
**W-5060 Bergisch Gladbach 2(DE)**

## (54) Verfahren zur Herstellung von Thionocarbamaten.

(57) Ein vorteilhaftes Verfahren zur Herstellung von Thionocarbamaten durch Umsetzung von Xanthogenaten mit Aminen in Gegenwart von Oxidationsmitteln ist dadurch gekennzeichnet, daß man es in einem Zweiphasensystem aus Wasser und einem organischen Lösungsmittel durchführt.

EP 0 416 364 A1

## VERFAHREN ZUR HERSTELLUNG VON THIONOCARBAMATEN

Die vorliegende Erfindung betrifft ein verbessertes Verfahren zur Herstellung von Thionocarbamaten aus Alkalixanthogenaten und Aminen in Gegenwart eines Oxidationsmittels. Thionocarbamate werden als Flotationshilfsmittel, z.B. bei der Anreicherung von Kupfererzen, verwendet (siehe z.B. US-PS 2 691 635 und US-PS 3 975 264).

Es ist bekannt Thionocarbamate herzustellen, indem man Xanthogenate mit Aminen in Gegenwart eines Oxidationsmittels umsetzt (siehe EP-OS 0 024 530). Nachteilig bei diesem Verfahren ist, daß es insbesondere beim Einsatz von Aminen mit kurzen Alkylresten Thionocarbamate in nicht zufriedenstellenden Ausbeuten liefert.

Es wurde nun ein Verfahren zur Herstellung von Thionocarbamaten durch Umsetzung von Xanthogenaten mit Aminen in Gegenwart von Oxidationsmitteln gefunden, das dadurch gekennzeichnet ist, daß man die Reaktion in einem Zweiphasensystem aus Wasser und einem organischen Lösungsmittel durchführt.

Als organische Lösungsmittel kommen generell alle Lösungsmittel in Frage, die mit Wasser schlecht oder nicht mischbar sind und ein ausreichendes Lösungsvermögen für das jeweils eingesetzte Amin und das entstehende Thionocarbamat aufweisen.

Geeignete organische Lösungsmittel sind beispielsweise aliphatische, cycloaliphatische und aromatische Kohlenwasserstoffe, halogenierte, aliphatische und aromatische Kohlenwasserstoffe, Ester, Ether, Nitroaromaten, Ketone, Schwefelkohlenstoff sowie beliebige Mischungen solcher Lösungsmittel.

Als Beispiele seien genannt: n-Pentan, n-Hexan, n-Heptan, Petrolether, Leichtbenzin, Cyclohexan, Dekalin, Benzol, Toluol, Xylol, Chlorbenzol, Nitrobenzol, 1,2,3,4-Tetrahydronaphthalin,o-Dichlorbenzol, Methylenchlorid, Chloroform, Essigsäuremethylester, Essigsäureethylester, Essigsäurepropylester, Essigsäurebutylester, Anisol, Diethylether, Diisopropylether, tert.-Butylmethylether und 4-Methyl-2-pentanon.

Besonders bevorzugt sind Essigsäuremethylester und Essigsäureethylester.

Das Volumenverhältnis von organischem Lösungsmittel zu Wasser kann in weiten Bereichen variiert werden, bei spielsweise von 100:1 bis 1:100. Bevorzugt liegt dieses Verhältnis im Bereich von 10:1 bis 1:10, besonders bevorzugt im Bereich 3:1 bis 1:3.

Das Gewichtsverhältnis von Lösungsmittelgemisch (= organisches Lösungsmittel und Wasser) zu Xanthogenat richtet sich nach der Löslichkeit der verwendeten Komponenten und kann beispielsweise im Bereich von 10:1 bis 1:1 liegen. Bevorzugt beträgt dieses Verhältnis 6:1 bis 3:1.

Als Amine können beispielsweise solche der Formel (I) eingesetzt werden

$R^1 - NH_2$ (I),

bei der

$R^1$ für einen geradkettigen oder verzweigten Alkylrest mit 1 bis 5 C-Atomen steht.

Bevorzugt steht R1 für Methyl, Ethyl, n-Propyl oder i-Propyl.

Die Dosierung der Amine bezogen auf das eingesetzte Xanthogenat ist in einem weiten Rahmen variierbar Üblicherweise wird das Amin in einem Überschuß eingesetzt. Der Überschuß kann beispielsweise 1 bis 100 Mol-% betragen. Bevorzugt ist ein Überschuß von 10 bis 50 Mol-%, bezogen auf das eingesetzte Xanthogenat.

Als Xanthogenate können beispielsweise solche der Formel (II) eingesetzt werden

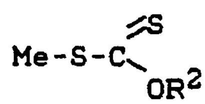

(II),

in der

Me für ein Alkalimetall und

$R^2$ für $C_1$-$C_{12}$-Alkyl, $C_3$-$C_{12}$-Cycloalkyl, $C_6$-$C_{20}$-Aryl oder $C_6$-$C_{20}$-Aralkyl

stehen. Bei den Alkylresten kann es sich dabei um verzweigte oder geradkettige Reste handeln. Vorzugsweise stehen Me für Natrium oder Kalium und $R^2$ für $C_1$-$C_8$-Alkyl oder $C_3$-$C_8$-Cycloalkyl. Besonders bevorzugt steht $R^2$ für $C_3$-$C_6$-Alkyl.

Als Oxydationsmittel kommen für das erfindungsgemäße Verfahren grundsätzlich alle gängigen Oxidationsmittel in Frage. Beispiele sind: Alkalihypochlorite, Wasserstoffperoxid, Chlor, Brom, Iod, sowie Sauerstoff und Luft in Gegenwart von Katalysatoren. Bevorzugt ist Natriumhypochlorit, insbesondere in Form von technischer Chlorbleichlauge.

Die Oxidationsmittel werden zweckmäßigerweise in einem Überschuß bezogen auf das eingesetzte Xanthogenat eingesetzt. Der Überschuß kann beispielsweise 1 bis 100 Mol-% betragen. Bevorzugt ist ein Überschuß von 10 bis 50 Mol-%, bezogen auf das eingesetzt Xanthogenat.

Man kann beispielsweise das Xanthogenat und das Amin in dem erfindungsgemäßen Lösungsmittelgemisch vorlegen und dann das Oxidationsmittel langsam zudosieren. Selbstverständlich kann die Zugabe der einzelnen Komponenten auch in anderer Reihenfolge vorgenommen werden.

Die Reaktion wird vorzugsweise bei niedriger Temperatur durchgeführt, beispielsweise im Bereich 0 bis 30° C. Bevorzugt sind Temperaturen im Bereich 10 bis 20° C.

Die Reaktionsdauer ist abhängig von der Reaktivität der verwendeten Ausgangsmaterialien und liegt im allgemeinen im Bereich von 0,5 bis 20 Stunden. Bevorzugt ist eine Reaktionsdauer von 3 bis 7 Stunden.

Der pH-Wert der Reaktionsmischung liegt beim erfindungsgemäßen Verfahren im allgemeinen deutlich im alkalischen Bereich. Bevorzugt sind pH-Werte im Bereich von 11 bis 14.

Es kann zweckmäßig sein, das erfindungsgemäße Verfahren in Gegenwart eines Emulgators oder eines Phasentransfer-Katalysators durchzuführen.

Die Aufarbeitung der Reaktionsmischung kann so erfolgen, daß man zunächst die wäßrige und die organische Phase voneinander trennt, die organische Phase gegebenenfalls mit einem üblichen Trocknungsmittel trocknet und das organische Lösungsmittel daraus abdestilliert. Das so erhaltene Rohprodukt ist von ausreichender Reinheit für die Anwendung als Flotationshilfsmittel. Es kann im Bedarfsfall durch Destillation gereinigt werden.

Mit dem erfindungsgemäßen Verfahren lassen sich insbesondere Thionocarbamate der Formel (III) erhalten

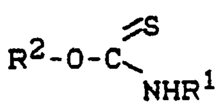 (III),

bei der $R^1$ die bei Formel (I) angegebene Bedeutung hat und $R^2$ die bei Formel (II) angegebene Bedeutung.

Das erfindungsgemäße Verfahren zeichnet sich überraschenderweise dadurch aus, daß mit ihm Thionocarbamate in deutlich verbesserten Ausbeuten erhalten werden können.

## Beispiele

### Beispiel 1

(Vergleichsbeispiel - Herstellung von Isopropyl-ethylthionocarbamat gemäß EP-OS 0 024 530)

179,54 g (1 Mol) Natriumisopropylxanthogenat (88 %ig) wurden in 380 ml Wasser gelöst. 118 ml 50 gew.-%ige Ethylaminlösung wurden innerhalb von 30 Minuten bei Raumtemperatur zugetropft. Anschließend wurden unter Kühlung bei 15° C 493 ml (1,13 Mol) technische Chlorlauge (Gehalt 2,3 Mol Hypochlorit/l) innerhalb von 3 Stunden zugetropft, wobei der pH-Wert bei etwa 12 gehalten wurde. Der Ansatz wurde weitere 3 Stunden bei 15° C gerührt. Der ausgefallene Schwefel wurde abfiltriert und die organische Phase abgetrennt. So wurden 120 g Produkt in Form eines gelben Öls erhalten. Das entspricht einer Ausbeute von 82 % der Theorie. Das Produkt war 97 %ig rein.

### Beispiel 2

(Herstellung von Isopropyl-ethyl-thionocarbamat - erfindungsgemäß)

179,54 g (1 Mol) Natriumisopropylxanthogenat (88 %ig) wurden in einem Gemisch aus 400 ml Wasser und 400 ml Essigsäureethylester gelöst. 118 ml (1,45 Mol) 70 gew.-%ige wäßrige Ethylaminlösung wurden innerhalb von 30 Minuten bei Raumtemperatur zugetropft. Anschließend wurde unter Kühlung bei 15° C 493 ml (1,13 Mol) technische Chlorlauge (Gehalt 2,3 Mol Hypochlorit/l) innerhalb von 3 Stunden zugetropft, wobei der pH-Wert bei etwa 12 gehalten wurde. Der Ansatz wurde weitere 3 Stunden bei 15° C gerührt. Der ausgefallene Schwefel wurde abfiltriert und die organische Phase abgetrennt. Aus der organischen Phase wurde das Lösungsmittel im Vakuum ausdestilliert. So wurden 139,6 g Produkt in Form eines Öls erhalten. Das entspricht einer Ausbeute von 95 % der Theorie. Das Produkt war 98 %ig rein.

### Beispiel 3

Es wurde verfahren wie in Beispiel 2, jedoch wurden nur 91,6 g (1,13 Mol) der Ethylaminlösung eingesetzt. Das Produkt entsprach qualitativ und quantitativ dem gemäß Beispiel 2 erhaltenen.

## Ansprüche

1. Verfahren zur Herstellung von Thionocarbamaten durch Umsetzung von Xanthogenaten mit Aminen in Gegenwart von Oxidationsmitteln, dadurch gekennzeichnet, daß man die Reaktion in einem Zweiphasensystem aus Wasser und einem organischen Lösungsmittel durchführt.

2. Verfahren nach Anspruch 1, dadurch gekennzeichnet, daß man als organisches Lösungsmittel aliphatische, cycloaliphatische oder aromatische Kohlenwasserstoffe, halogenierte aliphatische oder aromatische Kohlenwasserstoffe, Ester, Ether, Nitroaromaten, Ketone oder Mischungen solcher Lösungsmittel einsetzt.

3. Verfahren nach Ansprüchen 1 und 2, dadurch

gekennzeichnet, daß das Volumenverhältnis von organischem Lösungsmittel zu Wasser im Bereich 100:1 bis 1:100 liegt.

4. Verfahren nach Ansprüchen 1 bis 3, dadurch gekennzeichnet, daß das Gewichtsverhältnis von Lösungsmittelgemisch zu Xanthogenat im Bereich 10:1 bis 1:1 liegt.

5. Verfahren nach Ansprüchen 1 bis 4, dadurch gekennzeichnet, daß als Amine solche der Formel (I) eingesetzt werden

$R^1 - NH_2$ (I),

in der

$R^1$ für einen geradkettigen oder verzweigten Alkylrest mit 1 bis 5 C-Atomen steht.

6. Verfahren nach Ansprüchen 1 bis 5, dadurch gekennzeichnet, daß ein Xanthogenat der Formel (II) eingesetzt wird

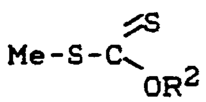 (II),

in der

Me für ein Alkalimetall und

$R^2$ für $C_1$-$C_{12}$-Alkyl, $C_3$-$C_{12}$-Cycloalkyl, $C_6$-$C_{20}$Aryl oder $C_6$-$C_{20}$-Aralkyl

stehen.

7. Verfahren nach Ansprüchen 1 bis 6, dadurch gekennzeichnet, daß man als Oxidationsmittel Alkalihypochlorite, Wasserstoffperoxid, Chlor, Brom, Iod oder Sauerstoff oder Luft in Gegenwart von Katalysatoren einsetzt.

8. Verfahren nach Ansprüchen 1 bis 7, dadurch gekennzeichnet, daß man die Reaktion bei Temperaturen im Bereich 0 bis 30° C durchführt.

9. Verfahren nach Ansprüchen 1 bis 8, dadurch gekennzeichnet, daß die Reaktionszeit zwischen 0,5 und 20 Stunden liegt.

10. Verfahren nach Ansprüchen 1 bis 9, dadurch gekennzeichnet, daß der pH-Wert während der Reaktion im Bereich 11 bis 14 gehalten wird.

Europäisches Patentamt

# EUROPÄISCHER RECHERCHENBERICHT

Nummer der Anmeldung

**EP 90 11 5969**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | FR-A-2 255 297 (HOECHST) --- | | C 07 C 333/04 |
| A | DE-A-2 353 976 (HOECHST) --- | | |
| D,A | EP-A-0 024 530 (LIGURCHIM) --- | | |
| D,A | US-A-3 975 264 (BOLTH et al.) ----- | | |

| RECHERCHIERTE SACHGEBIETE (Int. Cl.5) |
|---|
| C 07 C 333/00 |

Der vorliegende Recherchenbericht wurde für alle Patentansprüche erstellt

| Recherchenort | Abschlußdatum der Recherche | Prüfer |
|---|---|---|
| Den Haag | 30 November 90 | ZAROKOSTAS K. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument